(19) Europäisches Patentamt
European Patent Office
Office européen des brevets

(11) **EP 3 970 806 A1**

(12) **EUROPEAN PATENT APPLICATION**

(43) Date of publication:
**23.03.2022 Bulletin 2022/12**

(21) Application number: **21160322.0**

(22) Date of filing: **02.03.2021**

(51) International Patent Classification (IPC):
*A61Q 19/00* (2006.01)  *A61P 17/10* (2006.01)
*A61Q 17/00* (2006.01)  *A61K 8/365* (2006.01)
*A61K 8/42* (2006.01)  *A61K 8/44* (2006.01)
*A61K 8/37* (2006.01)  *A61K 8/92* (2006.01)
*A61K 31/19* (2006.01)  *A61K 31/17* (2006.01)
*A61K 31/195* (2006.01)  *A61K 31/215* (2006.01)

(52) Cooperative Patent Classification (CPC):
**A61Q 19/00; A61K 8/365; A61K 8/375; A61K 8/42;
A61K 8/44; A61K 8/92; A61K 31/17; A61K 31/19;
A61K 31/195; A61K 31/215; A61P 17/10;
A61Q 17/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.09.2020 EP 20197602**

(71) Applicant: **Beiersdorf AG
20253 Hamburg (DE)**

(72) Inventors:
• **HÜPEDEN, Jennifer
20251 Hamburg (DE)**

• **LASCHET, Mirja
20255 Hamburg (DE)**
• **TOM DIECK, Karen
22399 Hamburg (DE)**
• **FÖLSTER, Heike
22149 Hamburg (DE)**
• **SELLKAU, Sabine
22455 Hamburg (DE)**

(74) Representative: **Uexküll & Stolberg
Partnerschaft von
Patent- und Rechtsanwälten mbB
Beselerstraße 4
22607 Hamburg (DE)**

(54) **COMPOSITION FOR PROMOTING THE SKIN MICROBIOME**

(57) The present invention generally relates to the field of skin care. More particularly, the invention relates to a skin care composition that comprises growth-enhancing compounds which promote the development of a healthy skin microbiome. These growth-enhancing compounds are metabolized by the microorganisms of the skin microbiome after their topical application to the skin, thereby supporting growth of the microorganisms. The growth-enhancing compounds and compositions comprising same can be used for treating or preventing skin diseases, for promoting the development of a healthy skin microbiome, for stabilizing a healthy skin microbiome, and for promoting growth of bacteria of the skin microbiome.

Figure 3 A

EP 3 970 806 A1

**Description**

The present invention generally relates to the field of skin care. More particularly, the invention relates to a skin care composition that comprises growth-enhancing compounds which promote the development of a healthy skin microbiome. These growth-enhancing compounds are metabolized by the microorganisms of the skin microbiome after their topical application to the skin, thereby supporting growth of the microorganisms. The growth-enhancing compounds and compositions comprising same can be used for treating or preventing skin diseases, for promoting the development of a healthy skin microbiome, for stabilizing a healthy skin microbiome, and for promoting growth of bacteria of the skin microbiome.

BACKGROUND OF THE INVENTION

The human skin is colonized by millions of microorganisms, in particular bacteria and fungi. Most of these microorganisms are harmless or even beneficial to their host. The entirety of the microorganisms living on the human skin is referred to as the "skin microbiome". There is emerging evidence that the skin microbiome has a direct influence on human health. For example, bacteria of the skin microbiome contribute to health by secreting antimicrobial compounds that provide a barrier to infection of the body with pathogenic bacteria or fungi. In addition, the skin microbiome interacts with the immune system and effectively stimulates the immune system to combat pathogenic bacteria and fungi.

The human skin is a habitat that is generally unfavorable for microorganisms. The skin is salty, dry and continuously exposed to UV radiation. In addition, the availability of nutrients on the skin surface is limited to compounds that are released from dead skin cells of the upper epidermal layer. Due to these harsh conditions, the overall number of bacterial and fungal cells on skin is rather low, especially in dry areas such as the forearm, leg and lower back. Daily washing the skin with soap as well as the use of inappropiate cosmetic products provide additional antimicrobial effects which further reduce the overall number of bacterial and fungal cells on skin and may result in a disturbance of the skin microbiome.

In a healthy microbiome, there appears to be a balance of the resident microorganisms with respect to each other. A disturbance of said balance is thought to contribute to diseases or disorders, such as acne or eczema. In particular, if the number of bacteria from the resident flora, such as *Staphylococcus epidermidis*, becomes too low, there is a risk that the skin is colonized with pathogenic or potentially pathogenic bacteria, such as *Staphylococcus aureus*. A healthy and stable microbiome normally prevents the colonization of the skin with such pathogenic or potentially pathogenic bacteria. Accordingly, there is a need for means and methods that result in the maintainance of a healthy skin microbiome.

In addition, there is a need for means and methods that could promote growth of the resident microorganisms in the microbiome.

DESCRIPTION OF THE INVENTION

It has been found in the course of the present invention that it is possible to actively support a balanced microbiome by application of a skin care composition that comprises a high amount of compounds which can be metabolized by the microorganisms of the skin microbiome, thereby promoting their growth. In particular, it has been found that certain compounds, upon their topical application to the skin, can be used as carbon, nitrogen, or lipid source by the microorganisms of the skin microbiome. The topical application of such compounds therefore provides improved metabolic conditions for these microorganisms on the skin surface.

The growth-enhancing compounds disclosed herein are metabolized by the resident microorganisms, i.e. the microorganisms that normally occur as part of the human skin microbiome, much more effective compared to transient microorganisms which colonize the skin only temporarily, e.g. after touching contaminated objects with the hands. As a result, the compounds disclosed herein provide a growth advantage to resident microorganisms, such as S. *epidermidis*, over pathogenic microorganisms like S. *aureus*. In this way, these compounds assist in stabilizing and promoting growth of the resident microorganisms of the skin microbiome.

Accordingly, in a first aspect, the present invention relates to a skin care composition for promoting the skin microbiome, wherein said composition is formulated for topical application to the skin and comprises

(a) pyruvic acid, pyruvate, lactic acid, lactate or mixtures thereof as a carbon source.

Apart from the carbon source, the skin care composition of the present invention further comprises at least one of following components:

(b) urea and/or arginine as a nitrogen source;
(c) caprylic/capric triglycerides as a lipid source.

Hence, the invention relates to a composition for promoting the skin microbiome which comprises a carbon source, which either is pyruvic acid, pyruvate, lactic acid, lactate or mixtures thereof, e.g. a combination of pyruvate and lactate, and at least one additional growth-enhancing compound. The additional growth-enhancing compound can be a nitrogen source selected from urea and arginine or caprylic/capric triglycerides as a lipid source. In a particularly preferred embodiment, the composition of the invention comprises all three types of components. Accordingly, in a preferred aspect, the invention relates to a skin care composition for promoting the skin microbiome, wherein said composition is formulated for topical application to the skin and comprises

    (a) pyruvic acid, pyruvate lactic acid, lactate or mixtures thereof as a carbon source,
    (b) urea and/or arginine as a nitrogen source,
    (c) caprylic/capric triglycerides as a lipid source.

In a particularly preferred aspect, the invention relates to a skin care composition for promoting the skin microbiome, wherein said composition is formulated for topical application to the skin and comprises

    (a) pyruvic acid, pyruvate or mixtures thereof as a carbon source,
    (b) urea and/or arginine as a nitrogen source,
    (c) optionally, caprylic/capric triglycerides as a lipid source.

In one preferred embodiment of the invention, the composition of the invention comprises pyruvic acid, pyruvate or mixtures thereof as a carbon source. If pyruvic acid is used, it will normally dissociate to some extent in aqueous solution. Therefore, it will be present in an aqueous environment as a mixture of undissociated pyruvic acid and the corresponding salt pyruvate. As shown in the below examples, pyruvic acid and pyruvate are able to significantly increase the cell number of the resident bacteria when applied in the form of a skin care composition to the facial skin. As used herein, pyruvate refers to a salt or ester of 2-oxopropanoic acid. Preferably, the pyruvate in the composition is calcium or sodium pyruvate. Sodium pyruvate is used in some cosmetic compositions for stimulating collagen synthesis.

A preferred skin care composition of the invention comprises the following:

    (a) pyruvic acid, pyruvate or mixtures thereof as a carbon source,
    (b) urea as a nitrogen source, and
    (c) as an optional component, caprylic/capric triglycerides as a lipid source.

It is preferred that the pyruvic acid, pyruvate or the mixtures of both components is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). An amount of 0.05 to 1.0% (w/w) is particularly preferred. In some embodiments, the amount of pyruvic acid, pyruvate or mixtures thereof present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). All weight percentages mentioned herein in relation to certain components refer, unless stated otherwise, to the total weight of the preparation or composition.

Similarly, it is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

This composition may optionally include caprylic/capric triglycerides as a lipid source. As used herein, the term "caprylic/capric triglycerides" refers to triglycerides in which the glycerol is esterified with the fatty acids caprylic acid and capric acid. The caprylic/capric triglycerides are preferably present in an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

Another preferred skin care composition of the invention comprises the following:

    (a) pyruvic acid, pyruvate or mixtures thereof as a carbon source,
    (b) arginine a as a nitrogen source, and
    (c) as an optional component, caprylic/capric triglycerides as a lipid source.

The pyruvic acid, pyruvate or the mixtures of both components is preferably present in the composition in an amount as outlined above. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

In another embodiment of the invention, the composition of the invention comprises lactic acid, lactate or mixtures thereof as a carbon source. Like pyruvic acid or pyruvate, lactic acid and lactate are able to increase the cell number of the resident bacteria when applied to the facial skin. As used herein, lactate refers to a salt or ester of lactic acid. If lactic acid is used, it will normally dissociate to some extent in aqueous solution. Therefore, it will be present in an aqueous environment as a mixture of undissociated lactic acid and the corresponding salt lactate. Preferably, the lactate in the composition is calcium or sodium lactate. Sodium lactate is used as a humectant in some cosmetic compositions as well as for pH stabilization. Preferred skin care compositions of the invention that comprise lactate as a growth-enhancing component, include:

A preferred skin care composition of the invention comprises the following:

(a) lactic acid, lactate or mixtures thereof as a carbon source,
(b) urea as a nitrogen source, and
(c) as an optional component, caprylic/capric triglycerides as a lipid source.

It is preferred that the lactic acid, lactate or the mixture of both components is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of lactic acid, lactate or the mixture of both components present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

Similarly, it is preferred that the urea is present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.7 to 2.0% (w/w), more preferably 0.8 to 1.5% (w/w), such as 1.0% (w/w). In some embodiments, the amount of urea present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w).

This composition may optionally include caprylic/capric triglycerides as a lipid source. The caprylic/capric triglycerides are preferably present in an amount of 0.1 to 6.0% (w/w), more preferably 0.2 to 6.0% (w/w), more preferably 0.3 to 5.5% (w/w), more preferably 0.4 to 5.0% (w/w), more preferably 0.5 to 4.5% (w/w), more preferably 0.6 to 4.0% (w/w), more preferably 0.7 to 3.5% (w/w), more preferably 0.8 to 3.0% (w/w), more preferably 0.9 to 2.5% (w/w), more preferably 1.0 to 3.0% (w/w), more preferably 1.5 to 2.5% (w/w), such as 2.0% (w/w). In some embodiments, the amount of caprylic/capric triglycerides present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), at least 3.0 (w/w), at least 4.0 (w/w), or at least 5.0 (w/w).

Another preferred skin care composition of the invention comprises the following:

(a) lactic acid, lactate or the mixture thereof as a carbon source,
(b) arginine a as a nitrogen source, and
(c) as an optional component, caprylic/capric triglycerides as a lipid source.

The lactic acid, lactate, or the mixture of both components is preferably present in the composition in an amount as outlined above. The arginine is preferably present in the composition in an amount of 0.05 to 5.0% (w/w), more preferably 0.1 to 5.0% (w/w), more preferably 0.2 to 4.5% (w/w), more preferably 0.3 to 4.0% (w/w), more preferably 0.4 to 3.5% (w/w), more preferably 0.5 to 3.0% (w/w), more preferably 0.6 to 2.5% (w/w), more preferably 0.8 to 2.0% (w/w), more preferably 0.9 to 1.5% (w/w), such as 1.0% (w/w). In some embodiments, the amount of arginine present in the composition is at least 0.3 (w/w), at least 0.4 (w/w), at least 0.5 (w/w), at least 0.6 (w/w), at least 0.7 (w/w), at least 0.8 (w/w), at least 0.9 (w/w), at least 1.0 (w/w), at least 2.0 (w/w), or at least 3.0 (w/w). This composition may optionally include caprylic/capric triglycerides as a lipid source. If present, the caprylic/capric triglycerides are preferably used in the amounts outlines above.

Particularly preferred compositions of the invention include, but are not limited to:

A skin care composition comprising:

(a) 0.05 to 5.0% (w/w) pyruvic acid, pyruvate or a mixture thereof as a carbon source,
(b) 0.05 to 5.0% (w/w) urea as a nitrogen source, and
(c) as an optional component, 0.05 to 6.0% (w/w) caprylic/capric triglycerides as a lipid source.

A skin care composition comprising:

(a) 0.05 to 5.0% (w/w) pyruvic acid, pyruvate or a mixture thereof as a carbon source,
(b) 0.05 to 5.0% (w/w) arginine as a nitrogen source, and
(c) as an optional component, 0.05 to 6.0% (w/w) caprylic/capric triglycerides as a lipid source.

A skin care composition comprising:

(a) 0.5 to 3.0% (w/w) pyruvic acid, pyruvate or a mixture thereof as a carbon source,
(b) 0.5 to 3.0% (w/w) urea as a nitrogen source, and
(c) as an optional component, 0.5 to 4.5% (w/w) caprylic/capric triglycerides as a lipid source.

A skin care composition comprising:

(a) 0.5 to 3.0% (w/w) pyruvic acid, pyruvate or a mixture thereof as a carbon source,
(b) 0.5 to 3.0% (w/w) arginine as a nitrogen source, and
(c) as an optional component, 0.5 to 4.5% (w/w) caprylic/capric triglycerides as a lipid source.

A skin care composition comprising:

(a) 1.0 to 1.5% (w/w) pyruvic acid, pyruvate or a mixture thereof as a carbon source,
(b) 1.0 to 1.5% (w/w) urea as a nitrogen source, and
(c) as an optional component, 1.5 to 2.5% (w/w) caprylic/capric triglycerides as a lipid source.

A skin care composition comprising:

(a) 1.0 to 1.5% (w/w) pyruvic acid, pyruvate or a mixture thereof as a carbon source,
(b) 1.0 to 1.5% (w/w) arginine as a nitrogen source, and
(c) as an optional component, 1.5 to 2.5% (w/w) caprylic/capric triglycerides as a lipid source.

A skin care composition comprising:

(a) 0.1 to 5.0% (w/w) lactic acid, lactate or a mixture thereof as a carbon source,
(b) 0.1 to 5.0% (w/w) urea as a nitrogen source, and
(c) as an optional component, 0.2 to 6.0% (w/w) caprylic/capric triglycerides as a lipid source.

A skin care composition comprising:

(a) 0.1 to 5.0% (w/w) lactic acid, lactate or a mixture thereof as a carbon source,
(b) 0.1 to 5.0% (w/w) arginine as a nitrogen source, and
(c) as an optional component, 0.2 to 6.0% (w/w) caprylic/capric triglycerides as a lipid source.

A skin care composition comprising:

(a) 0.5 to 3.0% (w/w) lactic acid, lactate or a mixture thereof as a carbon source,
(b) 0.5 to 3.0% (w/w) urea as a nitrogen source, and
(c) as an optional component, 0.5 to 4.5% (w/w) decaprylic/capric triglycerides as a lipid source.

A skin care composition comprising:

(a) 0.5 to 3.0% (w/w) lactic acid, lactate or a mixture thereof as a carbon source,
(b) 0.5 to 3.0% (w/w) arginine as a nitrogen source, and
(c) as an optional component, 0.5 to 4.5% (w/w) caprylic/capric triglycerides as a lipid source.

A skin care composition comprising:

(a) 1.0 to 1.5% (w/w) lactic acid, lactate or a mixture thereof as a carbon source,
(b) 1.0 to 1.5% (w/w) urea as a nitrogen source, and
(c) as an optional component, 1.5 to 2.5% (w/w) caprylic/capric triglycerides as a lipid source.

A skin care composition comprising:

(a) 1.0 to 1.5% (w/w) lactic acid, lactate or a mixture thereof as a carbon source,
(b) 1.0 to 1.5% (w/w) arginine as a nitrogen source, and
(c) as an optional component, 1.5 to 2.5% (w/w) caprylic/capric triglycerides as a lipid source.

In a preferred aspect of the invention, the skin care composition of the invention is formulated as a water-containing composition for topical application to the skin that has a particularly low osmolality. It has been found that it is possible to further support a healthy and stable microbiome by reducing osmolality. A high number of osmotically active solutes in the compositions leads to a high level of osmotic stress after application of the composition to the skin. After application to the skin, the water contained in the composition evaporates to a significant extent so that the remaining water on the skin surface comprises salts and other osmotically active components in highly concentrated form. These concentrated compounds are able to bind free water in the environment which is crucial for the bacterial metabolism and hence for viability of the bacteria. In addition, due to the hyperosmotic conditions on the skin surface, water from inside of the bacterial cells diffuses along the osmotic gradient to the outside. This severely impacts viability of the bacterial cells.

The osmolality of a solution quantifies the osmotically active particles or ions contained in a solution. In contrast to molarity, which determines the number of particles or ions in a volume of fluid, osmolality refers to the mass weight. It is normally expressed as mOsm/kg water or mmol/kg water. The actual osmolality of a composition can be determined in accordance with methods well-known in the art. For example, osmolality can be measured by using a freezing point depression osmometer or a vapour pressure depression osmometer. Since the freezing point of a water-based composition is depressed dependent on the number of osmotically active particles or ions contained, it is possible to measure osmolality by determining the freezing point of the composition.

Since measuring the actual osmolality is cumbersome, the osmolality of a composition is often determined in practice by calculation the "theoretical osmolality" based on the molar masses of the compounds contained in said composition and the amounts of the compounds in the composition. As used herein, the theoretical osmolality $b_{osm}$ indicates the molality of the osmotically active particles in a solution. It is calculated according to the formula:

$$ b_{\mathrm{osm}} = \frac{n_{\mathrm{osm}}}{m_{\mathsf{Solvent}}} $$

wherein $n_{osm}$ indicates the amount of osmotically active particles in moles and $m_{Solvent}$ indicates the mass of the solvent in kg.

According to the invention, the skin care composition has a theoretical osmolality of 4000 mOsm/kg water or less. Preferably, the composition has a theoretical osmolality of 3900 mOsm/kg water or less, 3800 mOsm/kg water or less, 3700 mOsm/kg water or less, 3600 mOsm/kg water or less, 3500 mOsm/kg water or less, 3400 mOsm/kg water or less, 3300 mOsm/kg water or less, 3200 mOsm/kg water or less, 3100 mOsm/kg water or less, or 3000 mOsm/kg water or less. Stated differently, it is particularly preferred that skin care composition has a theoretical osmolality in the range between 3000-4000 mOsm/kg water, more preferably between 3100-3900 mOsm/kg water, between 3200-3800 mOsm/kg water, between 3300-3700 mOsm/kg water, or between 3400-3600 mOsm/kg water.

The composition of the invention preferably is a water-containing composition. The water content of the composition preferably is 30% (w/w) water or more. This means that the composition of the invention preferably comprises 30% (w/w) water or more, 35% (w/w) water or more, 40% (w/w) water or more, 45% (w/w) water or more, 50% (w/w) water or more, 55% (w/w) water or more, 60% (w/w) water or more, 65% (w/w) water or more, 70% (w/w) water or more, 75% (w/w) water or more, 80% (w/w) water or more, or 85% (w/w) water or more. It is particularly preferred that the composition of the invention comprises at least 70% (w/w) or at least 75% (w/w) water.

The skin care composition of the present invention is preferably formulated as a ready-to-use composition which is suitable for direct topical administration to the skin. Such a composition may be provided in different forms, including, but not limited to, in the form of a gel, lotion, fluid, cream, ointment, solution, a water-based emulsion, or the like. In a particularly preferred embodiment, the skin care composition of the present invention is formulated as a gel or lotion.

To provide for a low level of osmolality, the skin care compositions of the invention will use only reduced amounts of compounds which, upon their dissolution in water, are osmotically active. In some embodiments, certain osmotically active compounds like NaCl, KCl, $CaCl_2$ or $MgCl_2$ are completely omitted.

In one embodiment, the amount of NaCl in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any NaCl.

In yet one embodiment, the amount of KCl in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any KCl.

In one embodiment, the amount of $CaCl_2$ in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any $CaCl_2$.

In one embodiment, the amount of $MgCl_2$ in the composition which is to be administered to the skin is less than 1% (w/w), less than 0.9% (w/w), less than 0.8% (w/w), less than 0.7% (w/w), less than 0.6% (w/w), less than 0.5% (w/w), less than 0.4% (w/w), less than 0.3% (w/w), less than 0.2% (w/w), or less than 0.1% (w/w). In a particularly preferred embodiment, the composition does not comprise any $MgCl_2$.

Another component that contributes to osmolality is glycerol. It is preferred according to the invention that the amount of glycerol in the composition which is to be administered to the skin is less than 10% (w/w), less than 9% (w/w), less than 8% (w/w), less than 7% (w/w), less than 6% (w/w), less than 5% (w/w), less than 4% (w/w), less than 3% (w/w), less than 2% (w/w), or less than 1% (w/w). In a particularly preferred embodiment, the composition does not comprise any glycerol.

In one embodiment, the skin care composition of the invention as defined elsewhere hereinabove comprises less than 0.5% (w/w) of NaCl and less than 5% (w/w) of glycerol. In one embodiment, the composition comprises less than 0.5% (w/w) of NaCl and no glycerol. In another embodiment, the composition comprises less than 5% (w/w) of glycerol and no NaCl.

In another embodiment, the skin care composition comprises less than 0.5% (w/w) of KCl and less than 5% (w/w) of glycerol. In one embodiment, the composition comprises less than 0.5% (w/w) of KCl and no glycerol. In another embodiment, the composition comprises less than 5% (w/w) of glycerol and no KCl.

In yet another embodiment, the skin care composition comprises less than 0.5% (w/w) of $CaCl_2$ and less than 5% (w/w) of glycerol. In one embodiment, the composition comprises less than 0.5% (w/w) of $CaCl_2$ and no glycerol. In another embodiment, the composition comprises less than 5% (w/w) of glycerol and no $CaCl_2$.

In yet another embodiment, the skin care composition comprises less than 0.5% (w/w) of $MgCl_2$ and less than 5% (w/w) of glycerol. In one embodiment, the composition comprises less than 0.5% (w/w) of $MgCl_2$ and no glycerol. In another embodiment, the composition comprises less than 5% (w/w) of glycerol and no $MgCl_2$.

A particularly preferred skin care composition of the invention therefore has an osmolality of 4000 mOsm/kg water or less and comprises

(a) pyruvic acid, pyruvate, lactic acid, lactate or a mixture thereof as a carbon source, preferably in an amount of 0.05 to 5.0% (w/w),
(b) at least 50% (w/w) water,
(c) less than 0.5% (w/w) of NaCl,
(d) less than 5% (w/w) of glycerol.

and at least one of the following:

(e) urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) pyruvic acid, pyruvate, lactic acid, lactate or a mixture thereof as a carbon source, preferably in an amount of 0.05 to 5.0% (w/w),
(b) at least 50% (w/w) water,
(c) less than 0.5% (w/w) of KCl,
(d) less than 5% (w/w) of glycerol.

and at least one of the following:

(e) urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) pyruvic acid, pyruvate, lactic acid, lactate or a mixture thereof as a carbon source, preferably in an amount of 0.05 to 5.0% (w/w),
(b) at least 50% (w/w) water,
(c) less than 0.5% (w/w) of $CaCl_2$,
(d) less than 5% (w/w) of glycerol.

and at least one of the following:

(e) urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) pyruvic acid and/or pyruvate as a carbon source, preferably in an amount of 0.05 to 5.0% (w/w),
(b) at least 50% (w/w) water,
(c) less than 0.5% (w/w) of NaCl,
(d) less than 5% (w/w) of glycerol.

and at least one of the following:

(e) urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) pyruvic acid and/or pyruvate as a carbon source, preferably in an amount of 0.05 to 5.0% (w/w),
(b) at least 50% (w/w) water,
(c) less than 0.5% (w/w) of KCl,
(d) less than 5% (w/w) of glycerol.

and at least one of the following:

(e) urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) pyruvic acid and/or pyruvate as a carbon source, preferably in an amount of 0.05 to 5.0% (w/w),
(b) at least 50% (w/w) water,
(c) less than 0.5% (w/w) of $CaCl_2$,
(d) less than 5% (w/w) of glycerol.

and at least one of the following:

(e) urea and/or arginine as a nitrogen source, preferably in an amount of 0.05 to 5.0% (w/w),
(f) caprylic/capric triglycerides as a lipid source preferably in an amount of 0.05 to 6.0% (w/w).

A particularly preferred skin care composition of the invention therefore has an osmolality of 4000 mOsm/kg water or less and comprises

(a) 0.05 to 5.0% (w/w) pyruvic acid, pyruvate or a mixture thereof as a carbon source,
(b) 0.05 to 5.0% (w/w) urea as a nitrogen source,
(c) at least 50% (w/w) water,
(d) less than 0.5% (w/w) of NaCl,
(e) less than 5% (w/w) of glycerol, and
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.05 to 5.0% (w/w) pyruvic acid, pyruvate or a mixture thereof as a carbon source,
(b) 0.05 to 5.0% (w/w) arginine as a nitrogen source,
(c) at least 50% (w/w) water,
(d) less than 0.5% (w/w) of NaCl,
(e) less than 5% (w/w) of glycerol, and
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) pyruvic acid or pyruvate or a mixture thereof as a carbon source,
(b) 0.5 to 3.0% (w/w) urea as a nitrogen source,
(c) at least 50% (w/w) water,
(d) less than 0.5% (w/w) of NaCl,
(e) less than 5% (w/w) of glycerol, and
(f) as an optional component, 0.5 to 4.5% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) pyruvic acid or pyruvate or a mixture thereof as a carbon source,
(b) 0.5 to 3.0% (w/w) arginine as a nitrogen source,
(c) at least 50% (w/w) water,
(d) less than 0.5% (w/w) of NaCl,
(e) less than 5% (w/w) of glycerol, and
(f) as an optional component, 0.5 to 4.5% (w/w) caprylic/capric triglycerides as a lipid source.

Another particularly preferred skin care composition of the invention therefore has an osmolality of 4000 mOsm/kg water or less and comprises

(a) lactic acid and/or lactate as a carbon source, preferably in an amount of 0.05 to 5.0% (w/w),
(b) at least 50% (w/w) water,
(c) less than 0.5% (w/w) of NaCl,
(d) less than 5% (w/w) of glycerol.

and at least one of the following:

(e) urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) lactic acid and/or lactate as a carbon source, preferably in an amount of 0.05 to 5.0% (w/w),
(b) at least 50% (w/w) water,
(c) less than 0.5% (w/w) of KCl,
(d) less than 5% (w/w) of glycerol.

and at least one of the following:

(e) urea and/or arginine as a nitrogen source, preferably in an amount of 0.05 to 5.0% (w/w),
(f) caprylic/capric triglycerides as a lipid source preferably in an amount of 0.05 to 6.0% (w/w).

Another particularly preferred skin care composition of the invention therefore has an osmolality of 4000 mOsm/kg water or less and comprises

(a) lactic acid and/or lactate as a carbon source, preferably in an amount of 0.05 to 5.0% (w/w),
(b) at least 50% (w/w) water,
(c) less than 0.5% (w/w) of $CaCl_2$,
(d) less than 5% (w/w) of glycerol.

and at least one of the following:

(e) urea and/or arginine as a nitrogen source, preferably in an amount of 0.05 to 5.0% (w/w),
(f) caprylic/capric triglycerides as a lipid source preferably in an amount of 0.05 to 6.0% (w/w).

A particularly preferred skin care composition of the invention therefore has an osmolality of 4000 mOsm/kg water or less and comprises

(a) 0.05 to 5.0% (w/w) lactic acid or lactate or a mixture thereof as a carbon source,
(b) 0.05 to 5.0% (w/w) urea as a nitrogen source,
(c) at least 50% (w/w) water,
(d) less than 0.5% (w/w) of NaCl,
(e) less than 5% (w/w) of glycerol, and
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.05 to 5.0% (w/w) lactic acid or lactate or a mixture thereof as a carbon source,
(b) 0.05 to 5.0% (w/w) arginine as a nitrogen source,
(c) at least 50% (w/w) water,
(d) less than 0.5% (w/w) of NaCl,
(e) less than 5% (w/w) of glycerol, and
(f) as an optional component, 0.05 to 6.0% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) lactic acid or lactate or a mixture thereof as a carbon source,
(b) 0.5 to 3.0% (w/w) urea as a nitrogen source,
(c) at least 50% (w/w) water,
(d) less than 0.5% (w/w) of NaCl,
(e) less than 5% (w/w) of glycerol, and
(f) as an optional component, 0.5 to 4.5% (w/w) caprylic/capric triglycerides as a lipid source.

A particularly preferred skin care composition of the invention therefore has an osmolality of 4000 mOsm/kg water or less and comprises

(a) 0.5 to 3.0% (w/w) lactic acid or lactate or a mixture thereof as a carbon source,
(b) 0.5 to 3.0% (w/w) arginine as a nitrogen source,
(c) at least 50% (w/w) water,
(d) less than 0.5% (w/w) of NaCl,
(e) less than 5% (w/w) of glycerol, and
(f) as an optional component, 0.5 to 4.5% (w/w) caprylic/capric triglycerides as a lipid source.

The skin care composition of the present invention can further comprise additional compounds which are normally present in topical skin compositions. For example, the skin care composition of the present invention can further comprise at least one compound selected from the group consisting of perfumes, emollients, pigments, thickener, fillers, colorants, antioxidants, surfactants, lubricants, stabilizers, preservatives, solubilizers, emulsifiers and combinations of any of these.
The skin care compositions described herein are particularly useful for promoting and maintaining a healthy skin microbiome. The compositions preferably increase the overall number of microorganisms, more preferably the overall number of bacteria, in a skin areal to which the skin care compositions are applied. It is particularly preferred that the overall number of microorganisms, more preferably the overall number of bacteria, is increased after daily topical application of the composition over a period of 2-4 weeks by at least 2.5%, by at least 5%, by at least 7.5%, by at least 10%, by at least 12.5%, or by at least 15% relative to an untreated areal of the part of the body. It is particularly preferred that the number of bacteria of the species S. *epidermidis* is increased after daily topical application of the composition over a period of 2-4 weeks. Preferably, the number of S. *epidermidis* is increased by at least 2.5%, by at least 5%, by at least 7.5%, by at least 10%, by at least 12.5%, or by at least 15% relative to an untreated areal of the part of the body.
The compositions of the invention are useful for therapeutic purposes, in particular for preventing or treating skin diseases like acne. Hence, in another aspect the invention provides a skin care composition as defined hereinabove for use in a method of treating a skin disease, preferably acne. In one embodiment, the skin care composition is used for preventing the formation of acne. In another embodiment, the skin care composition is used for treating an acute state of acne. In yet another preferred embodiment, the skin care composition is used for preventing the reoccurrence of acne in a subject who has received a standard acne treatment. It is particularly preferred that the subject is a human.
The invention also provides methods for treating the skin of a subject by administering a skin care composition as described hereinabove. In one aspect, a method of treating a skin disease, preferably acne, is provided, said method comprising the topical administration of a skin care composition as described hereinabove.
Apart from therapeutic uses, the compositions of the invention can also be used for non-therapeutic, cosmetic purposes, e.g. for promoting the development of or stabilizing a healthy skin microbiome, for improving the appearance of the skin of a subject, for maintaining healthy skin, or for promoting bacterial growth of the skin microbiome. Accordingly, the present invention also relates to the use, preferably the non-therapeutic use, of a composition comprising

(a) a carbon source selected from the group consisting of pyruvic acid, pyruvate, lactic acid and/or lactate;
(b) a nitrogen source selected from the group consisting of urea and arginine; and/or
(c) caprylic/capric triglycerides

for promoting the development of or stabilizing a healthy skin microbiome or for promoting bacterial growth of the skin microbiome. In one embodiment, pyruvic acid, pyruvate or a mixture thereof is used as a carbon source for promoting the development of or stabilizing a healthy skin microbiome or for promoting bacterial growth of the skin microbiome. The pyruvate can be, for example, calcium or sodium pyruvate. More preferably, the pyruvic acid, pyruvate or a mixture thereof is used in combination with urea and/or arginine as a nitrogen source for promoting the development of or stabilizing a healthy skin microbiome or for promoting bacterial growth of the skin microbiome. Most preferably, the pyruvic acid, pyruvate or a mixture thereof is used in combination with urea and/or arginine as a nitrogen source and caprylic/capric triglycerides as a lipid source for promoting the development of or stabilizing a healthy skin microbiome or for promoting bacterial growth of the skin microbiome.

In another embodiment, lactate is used as a carbon source for promoting the development of or stabilizing a healthy skin microbiome or for promoting bacterial growth of the skin microbiome. The lactate can be, for example, calcium or sodium lactate. More preferably, the lactate is used in combination with urea and/or arginine as a nitrogen source for promoting the development of or stabilizing a healthy skin microbiome or for promoting bacterial growth of the skin microbiome. Most preferably, the lactate is used in combination with urea and/or arginine as a nitrogen source and caprylic/capric triglycerides as a lipid source for promoting the development of or stabilizing a healthy skin microbiome or for promoting bacterial growth of the skin microbiome.

## BRIEF DESCRIPTION OF THE FIGURE

Figure 1 shows the results from *in vitro* studies for promoting bacterial cell growth. It can be seen that the addition of pyruvate or lactate as a carbon source, either alone or in combination with a nitrogen source and/or a lipid source, induced a significant growth of S. *epidermidis* within 24 hours.

Figure 2 shows further results from *in vitro* studies for promoting bacterial cell growth. It can be seen that the addition of pyruvate, either alone or in combination with arginine, resulted in a significant growth of S. *epidermidis* within 24 hours.

Figure 3 shows the results from the analysis of skin hydration and cell count in a volunteer study. More specifically, Figure 3A shows the results from measuring skin hydration with a corneometer after application of compositions of the invention to the thigh skin for four weeks in an *in vivo* study. Figure 3B shows the results of the cell count measurements from the same study. It can be seen that the compositions of the invention led to significant skin hydration both after 2 and 4 weeks. Also, the compositions led to an increase in the cell number in the microbiome.

Figure 4 shows the results from 16S amplicon sequencing after applying the composition of the invention in vivo. No changes in the alpha and beta diversity were observed between the treatment group and the control group.

## EXAMPLES

The present invention is further illustrated by the following examples, which in no way should be construed as limiting. The entire contents of all of the references (including literature references, issued patents, published patent applications, and co pending patent applications) cited throughout this application are hereby expressly incorporated by reference.

Example 1: In vitro growth assays

Potential growth-enhancing compounds that are effective in the stabilization of the microbiome were tested by the following *in vitro* assay. Overnight cultures of S. *epidermidis* is inoculated in 20 ml CASO medium and incubated at 37°C. The culture was then diluted and adjusted to an OD600 of 0.25. From this suspension, the starting culture is prepared with a concentration of $5x \, 10^5$ CFU/ml by a 1:200 dilution in CASO deficiency medium. Subsequently, solutions containing the different active ingredients were mixed with the start cultures and incubated in a thermal mixer at 37°C by shaking. Samples are taken at the respective time points for testing, diluted with NaCl and plated out on CASO plates using the spiral plater. The plates are incubated for 24h at 37°C and then counted.

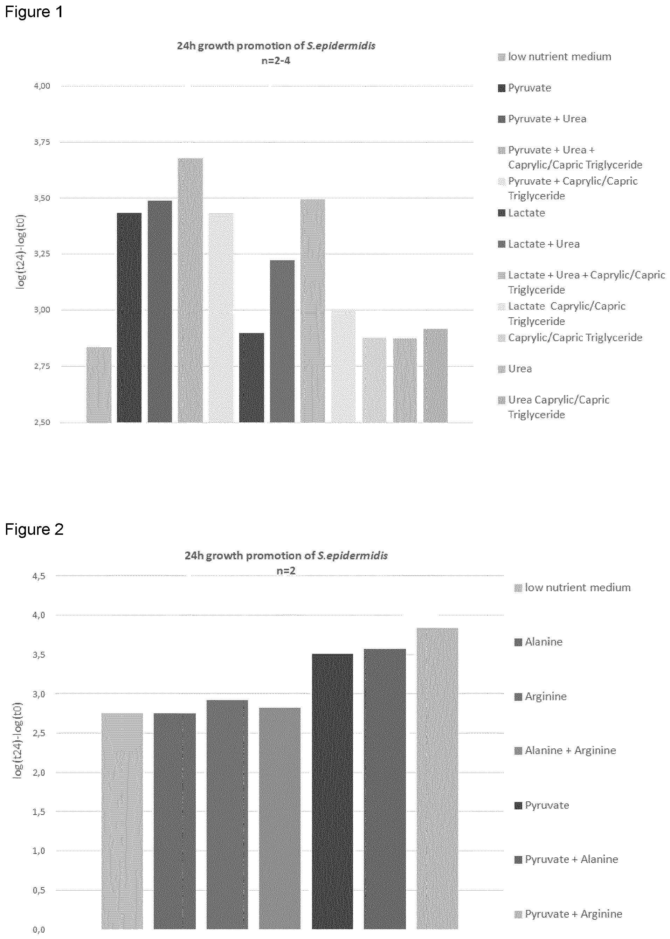

Results: The results can be shown in Figures 1 and 2. It can be taken from these figures that the addition of pyruvate or lactate to the low nutrient medium significantly promotes the growth of S. *epidermidis* within the 24 hours test period. The growth of the organism can even be further promoted by adding urea, argine or caprylic/capric triglycerides to the pyruvate or lactate.

Example 2: *In vivo* study

An in vivo study with 31 volunteers was conducted to examine whether the growth-enhancing compounds that were found effective in the *in vitro* test can also promote cell growth of the microorganisms in the skin microbiome *in vivo* when applied to the skin in the form of a lotion. For this purpose, a carrier lotion was prepared to which the test compounds, i.e. the compounds identified as growth-enhancing in the in *vitro* assays of Example 1, were added.

The composition of the carrier lotion is described in the following table.

Table 1: Composition of the carrier lotion

| INCI | wt. % |
|---|---|
| ethylhexyl salicylate | 5.00 |
| butyrospermum parkii butter | 4.50 |
| polysorbate 60 | 2.50 |
| glycerin | 1.50 |

(continued)

| INCI | wt. % |
|---|---|
| 4-methylbenzylidene camphor | 1.00 |
| glyceryl stearate | 1.00 |
| PEG-100 stearate | 1.00 |
| C12-15 alkyl benzoate | 1.00 |
| panthenol | 1.00 |
| phenoxyethanol | 0.70 |
| butyl methoxydibenzoylmethane | 0.60 |
| oryza sativa starch | 0.50 |
| hydroxyacetophenone | 0.40 |
| ethylhexylglycerin | 0.30 |
| tocopheryl acetate | 0.10 |
| tetrasodium glutamate diacetate | 0.10 |
| aqua | ad 100% |

The test formulations were prepared by mixing the compounds to be tested into the carrier lotion. The test formulations were composed as follows:

> Formulation 20: Carrier lotion + 1% urea, 0.3% pyruvate, 1% caprylic/capric triglycerides
> Formulation 30: Carrier lotion + 1% urea, 1% sodium Lactate
> Formulation 40: Carrier lotion + 1% urea, 1% sodium Lactate, 1% caprylic/capric triglycerides

For preconditioning, the study participants ceased to cream the thighs or wash them with shower gel or soap 1 week before the start of the study. 4 test areas were marked on the thighs of each participant (01 untreated control / test formulation 20 / test formulation 30 / test formulation 40). The above test formulations were applied two times a day within the marked areas of the thighs in the morning and evening by the test person at home. After 2 weeks and after 4 weeks, the hydration of the skin in the test areas was measured and samples of the microbiome were collected by swabbing on the thighs.

Example 3: Test area and sample analysis

The microbiome samples obtained from the *in vivo* study of Example 2 were analyzed by microbial colony count and bacterial diversity via 16S rRNA amplicon sequencing. In addition, the tested skin areal was analyzed by measuring skin hydration.

For measuring skin hydration, the skin areas of the study participants were measured with a Corneometer CM 825 (Courage & Khazaka, Cologne, Germany) using 5 repeated measurements. Skin hydration was expressed as permittivity in arbitrary units [a. u.], wherein an increase indicates an improved skin hydration.

For measuring the microbial colony count, the test site was rinsed with swabs (FLOQSwabs, Hain Lifescience GmbH, Nehren, Germany) by 3 x 10 lanes using 2 ml rinsing buffer (12,49 g/L $Na_2HPO$ ; 0,63 g/L $KH_2PO_4$ in molecular grade $H_2O$) in 15 ml Falcon tubes. The sample material was then plated within 60 minutes after collection on Columbia-Sheep-Blood-Tween (COST) agar plates and subsequently incubated. Specifically, 10 $\mu$l of the rinsing buffer was diluted in 990 $\mu$l 0.9% NaCl solution (1:100), and 100 $\mu$l of this dilution as well as 100 $\mu$l of the undiluted rinse was placed on COST plates with the spiral plater and subsequently incubated at 37°C for 24 h. After incubation, the bacterial colonies on agar plates were counted using the Countermat Flash.

For the molecular 16S rRNA analysis, the microorganisms obtained by rinsing the test site were subjected to the DNA extraction, 16S rRNA amplicon sequencing and subsequent bio-informatics analysis. The aim of this analysis was to detect changes in the bacterial diversity compared to baseline which would indicate an influence of the test formulation on the on the composition of the skin microbiome.

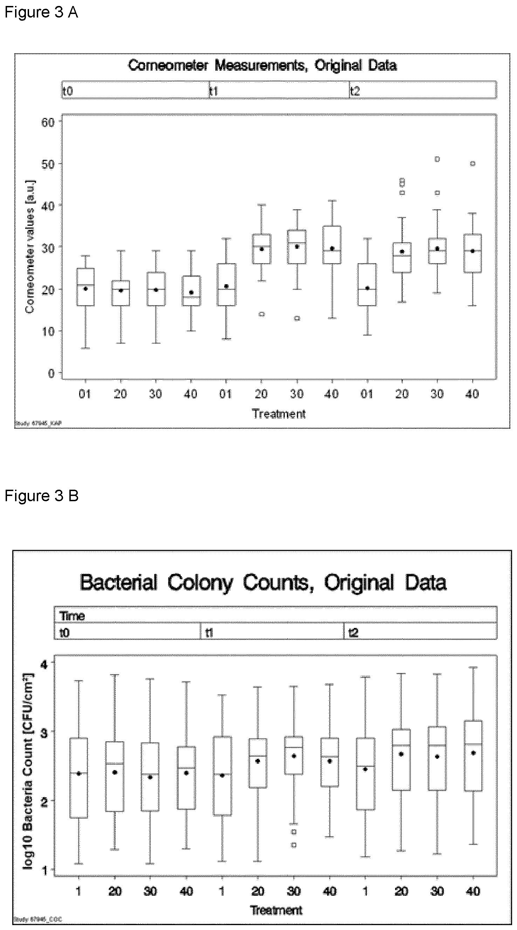

Results: The results of skin hydration measurements are depicted in Figure 3. As can be seen in Figure 3A, the test formulations provide for a significant skin hydration both after 2 and 4 weeks. In addition, as shown in Figure 3B, the test formulations led to significant increase in the cell count.

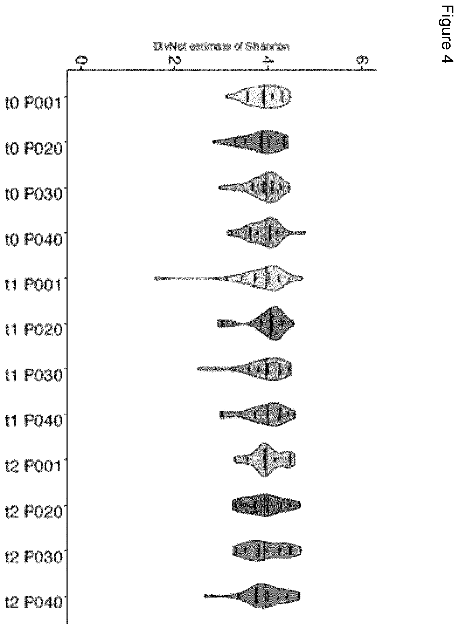

Figure 4 shows the results from 16S amplicon sequencing. No evident changes in the alpha and beta diversity were observed between the treatment group and the control group. Accordingly, the test formulations stabilize the skin microbiome and promote growth of the bacteria in the skin microbiome without changing the composition of the microbiome.

**Claims**

1. Skin care composition for promoting the skin microbiome, wherein said composition is formulated for topical application to the skin and comprises

   (a) lactic acid, lactate or a mixture thereof as a carbon source;
   (b) urea as a nitrogen source;
   (c) caprylic/capric triglycerides.

2. Skin care composition for promoting the skin microbiome according to claim 1, comprising

   (a) 0.5 to 3.0% (w/w) lactic acid, lactate or a mixture thereof as a carbon source;
   (b) 0.1 to 3.0% (w/w) urea as a nitrogen source;
   (c) 0.5 to 4.5% (w/w) caprylic/capric triglycerides.

**3.** Skin care composition for promoting the skin microbiome according to any of claims 1-2, further comprising at least one compound selected from the group consisting of a perfume, an emollient, a pigment, a thickener, a filler, a colorant, an antioxidant, a surfactant, a lubricant, a stabilizer, a preservative, a solubilizer, an emulsifier or a combination of any of these.

**4.** Skin care composition for promoting the skin microbiome according to any of claims 1-3 for use in a method of treating a skin disease,

**5.** Skin care composition for promoting the skin microbiome for use in a method of claim 4, wherein said skin disease is acne.

**6.** Non-therapeutic use of a composition comprising

(a) lactic acid, lactate or a mixture thereof as a carbon source;
(b) urea as a nitrogen source;
(c) caprylic/capric triglycerides

for promoting the development of or stabilizing a healthy skin microbiome.

**7.** Non-therapeutic use of a composition comprising

(a) lactic acid, lactate or a mixture thereof as a carbon source;
(b) urea as a nitrogen source;
(c) caprylic/capric triglycerides

for promoting bacterial growth of the skin microbiome.

**8.** Non-therapeutic use of any of claims 6-7, wherein the composition comprises

(a) 0.5 to 3.0% (w/w) lactic acid, lactate or a mixture thereof as a carbon source;
(b) 0.1 to 3.0% (w/w) urea as a nitrogen source;
(c) 0.5 to 4.5% (w/w) caprylic/capric triglycerides.

## Figure 1

24h growth promotion of *S.epidermidis*
n=2-4

Legend:
- low nutrient medium
- Pyruvate
- Pyruvate + Urea
- Pyruvate + Urea + Caprylic/Capric Triglyceride
- Pyruvate + Caprylic/Capric Triglyceride
- Lactate
- Lactate + Urea
- Lactate + Urea + Caprylic/Capric Triglyceride
- Lactate Caprylic/Capric Triglyceride
- Caprylic/Capric Triglyceride
- Urea
- Urea Caprylic/Capric Triglyceride

## Figure 2

24h growth promotion of *S.epidermidis*
n=2

Legend:
- low nutrient medium
- Alanine
- Arginine
- Alanine + Arginine
- Pyruvate
- Pyruvate + Alanine
- Pyruvate + Arginine

Figure 3 A

Figure 3 B

Figure 4

Europäisches Patentamt

European Patent Office

Office européen des brevets

# EUROPEAN SEARCH REPORT

**Application Number**

EP 21 16 0322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE GNPD [Online] MINTEL; 4 December 2018 (2018-12-04), anonymous: "Hand Cream", XP55838739, Database accession no. 6177215 * abstract * | 1,3,6,7 | INV. A61Q19/00 A61P17/10 A61Q17/00 A61K8/365 A61K8/42 A61K8/44 A61K8/37 |
| X | DATABASE GNPD [Online] MINTEL; 14 April 2016 (2016-04-14), anonymous: "Hand Rescue Intensive Regenerating Cream S.O.S for Hands", XP055838761, Database accession no. 3929737 * abstract * | 1,3,6,7 | A61K8/92 A61K31/19 A61K31/17 A61K31/195 A61K31/215 |
| X | DATABASE GNPD [Online] MINTEL; 28 August 2019 (2019-08-28), anonymous: "Pore Perfecting & Refining Serum", XP055838769, Database accession no. 6786219 * abstract * | 1,3-7 | |
| X | US 2015/118173 A1 (FARWICK MIKE [DE] ET AL) 30 April 2015 (2015-04-30) | 1-8 | TECHNICAL FIELDS SEARCHED (IPC) A61Q A61P A61K |
| Y | * paragraphs [0004], [0005], [0069] * | 1-8 | |
| X | Anon: "LACTIL", , 30 April 2012 (2012-04-30), XP055838827, Retrieved from the Internet: URL:http://glenncorp.com/wp-content/uploads/2013/11/DS_Lactil.pdf [retrieved on 2021-09-07] | 1-3,6-8 | |
| Y | * pages 1,2 * | 1-8 | |

The present search report has been drawn up for all claims

| Place of search | Date of completion of the search | Examiner |
|---|---|---|
| Munich | 13 September 2021 | Skulj, Primoz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 16 0322

This annex lists the patent family members relating to the patent documents cited in  the above-mentioned European search report.
The members are as contained in the European Patent Office EDP file on
The European Patent Office is in no way liable for these particulars which are merely given for the purpose of information.

13-09-2021

| Patent document cited in search report | Publication date | Patent family member(s) | Publication date |
|---|---|---|---|
| US 2015118173 A1 | 30-04-2015 | BR 112014026906 A2 | 11-07-2017 |
| | | CN    104284651 A | 14-01-2015 |
| | | DE 102012206999 A1 | 31-10-2013 |
| | | EP     2841050 A1 | 04-03-2015 |
| | | JP   2015520735 A | 23-07-2015 |
| | | US   2015118173 A1 | 30-04-2015 |
| | | WO   2013160065 A1 | 31-10-2013 |

EPO FORM P0459

For more details about this annex : see Official Journal of the European Patent Office, No. 12/82